# EUROPÄISCHE PATENTSCHRIFT

(11) **EP 0 888 789 B1**
(45) Veröffentlichungstag und Bekanntmachung des Hinweises auf die Patenterteilung: **25.09.2002**
(21) Anmeldenummer: 98111935.7
(22) Anmeldetag: 27.06.1998
(51) Int. Cl.: A61M 1/02

(54) **Steriles System und Verfahren zum Filtrieren von biologischen oder medizinischen Flüssigkeiten, insbesondere von Blut oder Blutkomponenten**
Sterile system and method for filtering biological or medical fluids, especially blood or blood components
Système stérile et procédé pour la filtration de liquides biologiques et médicaux, particulierement le sang et les composants sanguins

(30) Priorität: 01.07.1997 IT TO970573
(43) Veröffentlichungstag der Anmeldung: 07.01.1999
(73) Patentinhaber: BIOFIL S.r.l., I-41030 Villafranca di Medolla (MO) (IT)
(72) Erfinder: Mari, Giorgio, Dr., 41037 Mirandola (MO) (IT); Ganzerli, Federico, 41033 Concordia sulla Secchia (MO) (IT)
(74) Vertreter: Luderschmidt, Schüler & Partner GbR

(56) Entgegenhaltungen:
- EP-A- 0 642 801
- WO-A-91/17809
- WO-A-94/05344

## Beschreibung

Die Erfindung betrifft ein steriles System zum Filtrieren von biologischen oder medizinischen Flüssigkeiten, insbesondere von Blut oder Blutkomponenten sowie ein Verfahren zum Filtrieren von biologischen oder medizinischen Flüssigkeiten.

Zur Behandlung von Patienten werden bei bestimmten Krankheiten bestimmte Blutkomponenten benötigt, die dem Patienten zugeführt werden. So werden beispielsweise zur Behandlung von thrombozytogenischen Patienten Thrombozytenkonzentrate benötigt, die in einem extrakorporalen Kreislauf aus dem Blut eines Spenders gewonnen werden. Das dem Patienten zugeführte Thrombozytenkonzentrat sollte keine Leukozyten enthalten, da diese Immunreaktionen auslösen können. Lymphozyten, eine Unterfraktion der Leukozyten, die maßgebend für die Auslösung der Abwehrmechanismen sind, und die Thrombozyten lassen sich aber aufgrund ihrer geringen Größenunterschiede durch Zentrifugieren nur schwer von einander trennen. Um eine Immunisierung des Patienten auf jeden Fall zu vermeiden, wird das gewonnene Thrombozytenkonzentrat zur Abtrennung der Leukozyten im allgemeinen zusätzlich filtriert. Zum Filtrieren des Thrombozytenkonzentrates sind Filtriersysteme bekannt, die zur einmaligen Verwendung bestimmt sind. Diese Systeme werden auch zur Entfernung von Leukozyten aus anderen Blutkomponenten oder aus Vollblut eingesetzt.

Die WO 91/17809 beschreibt ein Filtriersystem für biologische Flüssigkeiten, das einen Sammelbeutel zur Aufnahme der filtrierten Flüssigkeit umfaßt, der über eine Schlauchleitung, in die ein Filter geschaltet ist, mit einem die zu filtrierende Flüssigkeit enthaltenden Vorlagebeutel verbunden ist. Die zu filtrierende Flüssigkeit wird unter dem Einfluß der Schwerkraft aus dem Vorlagebeutel über die Schlauchleitung und den Filter in den Sammelbeutel überführt. Nachteilig ist, daß nach der Beendigung des Filtrationsvorganges, d.h. nachdem die Flüssigkeit aufgehört hat, in den Sammelbehälter zu fließen, noch Flüssigkeit in der Schlauchleitung und dem Filter verbleibt. Zur Rückgewinnung dieser Flüssigkeit ist stromauf des Filters an der Schlauchleitung ein Gaseinlaß und stromab des Filters ein Gasauslaß an der Schlauchleitung vorgesehen. Nach der Beendigung des Filtrationsvorganges wird über den Einlaß in das System Gas geleitet, das unter Verdrängung der in dem System befindlichen Flüssigkeit, wieder über den Auslaß abgeführt wird.

Bei einer bevorzugten Ausführungsform des bekannten Filtriersystem wird das nach Beendigung des Filtrationsvorganges in das System zu leitende Gas in einem flexiblen Beutel bereitgestellt, der über eine Schlauchleitung an dem Gaseinlaß angeschlossen ist. Die aus dem System abgeführte Luft wird in einem zweiten flexiblen Beutel gesammelt, der über eine zweite Schlauchleitung an dem Gasauslaß angeschlossen ist. Zur Rückgewinnung der in dem System befindlichen Flüssigkeit wird nach Beendigung des Filtrationsvorganges der erste Beutel zusammengepreßt, wodurch das Gas in das System unter Verdrängung der Flüssigkeit strömt.

Aus der EP 0 642 801 A1 ist eine Filtriervorrichtung bekannt, die einen Sammelbeutel und ein Schlauchleitungssystem mit einem Filter umfaßt. Der Beutel, der die zu filtrierende Flüssigkeit enthält, ist nicht Bestandteil der Vorrichtung. Das Schlauchleitungssystem der bekannten Filtriervorrichtung weist neben einer Schlauchleitung, in die der Filter geschaltet ist, noch eine Bypassleitung mit einem Rückschlagventil auf, die stromauf bzw. stromab des Filters von der Schlauchleitung abzweigt, so daß nach Beendigung des Filtrationsvorganges Luft aus dem Sammelbeutel in den die zu filtrierende Flüssigkeit enthaltenden Beutel strömen kann. Die ausgangsseitige Kammer des Filters weist eine zusätzliche Belüftungsöffnung auf, die über eine Schlauchleitung mit einer Tropfleitung verbunden ist, die in dem zum Filter führenden Schlauchleitungsabschnitt geschaltet ist.

Der Erfindung liegt die Aufgabe zugrunde, ein einfach zu handhabendes System zum Filtrieren von biologischen oder medizinischen Flüssigkeiten, insbesondere von Blut oder Blutkomponenten zu schaffen, dessen Leitungen und dessen Filter sich vollständig entleeren lassen, ohne daß Luft von außen dem System zugeführt werden müßte, wodurch die Sterilität des Systems verloren ginge. Eine weitere Aufgabe der Erfindung liegt darin, ein einfach durchzuführendes Verfahren zum Filtrieren von biologischen oder medizinischen Flüssigkeiten anzugeben, das die vollständige Überführung der Flüssigkeit aus einem Behältnis in ein anderes Behältnis über einen Filter unter sterilen Bedingungen erlaubt.

Die Lösung dieser Aufgabe erfolgt erfindungsgemäße mit den im Patentanspruch 1 bzw. 8 angegebenen Merkmalen.

Bei dem erfindungsgemäßen Filtriersystem bzw. Verfahren wird die bereits im System befindliche sterile Luft vor der Einleitung des Filtriervorgangs, d.h. bevor die Strömungsverbindung zu dem Sammelbehältnis freigegeben wird, gesammelt und nach Beendigung des Filtriervorgangs, d. h. wenn die Flüssigkeit aufgehört hat, in das Sammelbehältnis zu strömen, zur vollständigen Entleerung des Systems genutzt.

Das erfindungsgemäße Filtriersystem weist einen mit einer hydrophoben Membran verschlossenen ersten Luftein- bzw. -auslaß auf, der mit der ersten Filterkammer in Strömungsverbindung steht und einen mit einer hydrophoben Membran verschlossenen zweiten Luftein- bzw. -auslaß auf, der mit der zweiten Filterkammer in Strömungsverbindung steht. Der erste und der zweite Luftein- bzw. -auslaß sind in einem kollabierbaren Behältnis angeordnet, in dem die in dem System befindliche Luft gesammelt und zur vollständigen Entleerung desselben wieder dem System zugeführt wird. Da die Luft dem System nicht von außen zugeführt wird, ist die Sterilität des Systems sichergestellt. Vorzugsweise wird die in dem kollabierbaren Behältnis gesammelte Luft nach Beednigung des Filtrationsvorganges zunächst in die erste Filterkammer unter Entleerung derselben und erst dann in die zweite Filterkammer unter Entleerung derselben geleitet.

Da der erste und zweite Luftein- bzw. -auslaß mit hydrophoben Membranen verschlossen sind, kann die Flüssigkeit nicht in das kollabierbare Behältnis eindringen, ohne daß es erforderlich ist, zusätzliche Mittel zum Unterbrechen des Flüssigkeitsflusses vorzusehen, die die Handhabung des Systems erschweren würden.

Der erste Luftein- bzw. -auslaß steht über eine erste Ent- bzw. Belüftungsleitung mit der ersten Filterkammer in Strömungsverbindung und der zweite Luftein- bzw. -auslaß ist über eine zweite Ent- bzw. Belüftungsleitung an der zweiten Filterkammer angeschlossen. Die erste Ent- bzw. Belüftungsleitung kann dabei von der ersten Sammelleitung abzweigen oder direkt an der ersten Filterkammer angeschlossen sein. Das kollabierbare Behältnis ist vorzugsweise als flexibler Kunststoffbeutel ausgebildet, der mit den Ent- bzw. Belüftungsleitungen verschweißt ist. Zur Befestigung des Kunststoffbeutels ist dieser vorteilhafterweise mit einer Lasche versehen, durch die die erste Sammelleitung geführt ist. Bei dieser Ausführungsform kann zum Verschließen und Öffnen des zweiten Luftein- bzw. Auslasses eine einfache Schlauchklemme an der zweiten Ent- bzw. Belüftungsleitung vorgesehen sein. Es ist aber auch möglich, daß das kollabierbare Behältnis direkt an den Filter angeformt ist.

In einer weiteren Ausführungsform ist der Filter ein Filter zur Entfernung von Leukozyten. In die zweite Sammelleitung kann aber auch ein Filter zur Entfernung von anderen Blutkomponenten aus Vollblut oder anderen Komponenten aus anderen biologischen oder medizinischen Flüssigkeiten geschaltet sein.

Im folgenden wird ein Ausführungsbeispiel unter Bezugnahme auf die Zeichnung näher erläutert.

Es zeigen:
- Figur 1: eine bevorzugte Ausführungsform eines Filtriersystems zur Entfernung von Leukozyten aus einem Thrombozytenkonzentrat in schematischer Darstellung,
- Figur 2: einen Schnitt durch ein Leukozytenfilter für das Filtriersystem von Figur 1 und
- Figur 3: den Ausschnitt A von Figur 1 in vergrößerter Darstellung.

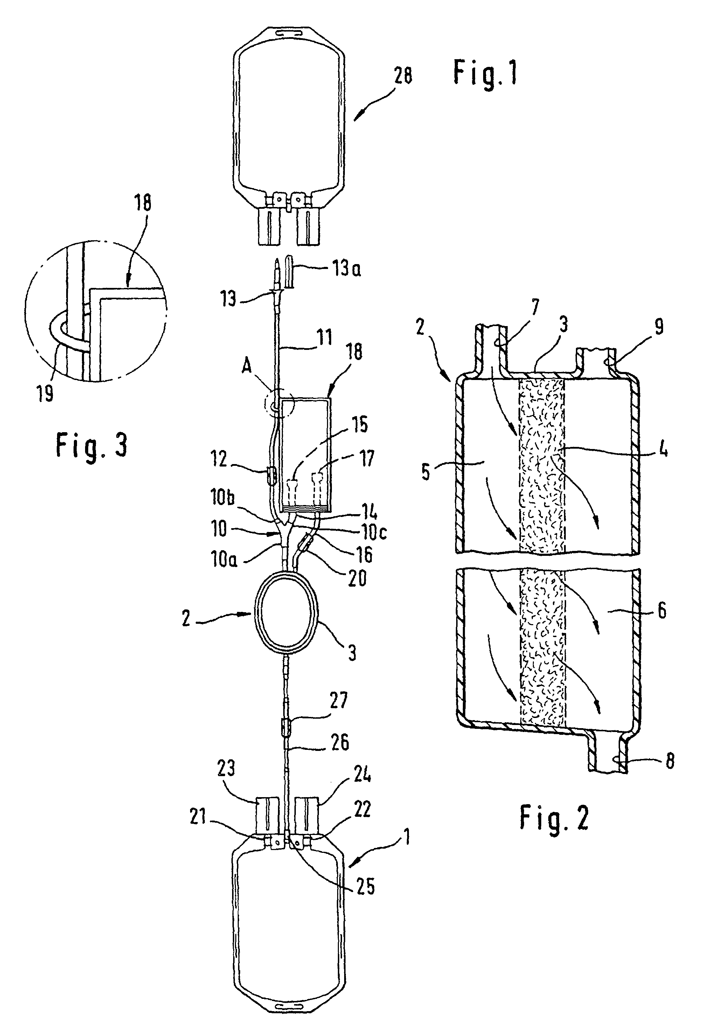

Figur 1 zeigt eine bevorzugte Ausführungsform des Filtriersystems in der Draufsicht. Das Filtriersystem umfaßt ein Sammelbehältnis 1 zur Aufnahme der filtrierten Flüssigkeit, in diesem Ausführungsbeispiel des leukozytenfreien Thrombozytenkonzentrats und einen Filter 2 zur Entfernung einer Blutkomponente, in diesem Ausführungsbeispiel einen Leukozytenfilter.

Der Leukozytenfilter 2 weist ein flaches Gehäuse 3 aufweist, das von einem Filtermaterial 4 in eine erste und zweite Filterkammer 5, 6 unterteilt ist (Figur 2). Die erste Filterkammer 5 weist an dem oberen Gehäuserand einen Einlaß 7 und die zweite Kammer 6 an dem unteren Gehäuserand einen Auslaß 8 und an dem oberen Gehäuserand einen Ent- bzw. Belüftungsanschluß 9 auf.

Der Einlaß 7 der ersten Filterkammer 5 ist mit dem gemeinsamen Anschlußstück 10a eines Abzweigungselements 10 verbunden. An dem einen Abzweigungsstück 10a des Abzweigungselements 10 ist das eine Ende einer ersten Sammelleitung 11 in Form einer flexiblen Schlauchleitung angeschlossen. Zum Öffnen und Schließen der ersten Sammelleitung 11 ist eine auf der Schlauchleitung sitzende Schlauchklemme 12 vorgesehen. Ein Spike-Konnektor 13 ist an dem freien Ende der ersten Sammelleitung befestigt, der mit einer Verschlußkappe 13a steril verschlossen ist. In Figur 1 ist der Spike-Konnektor mit abgenommener Verschlußkappe dargestellt.

Das andere Abzweigungsstück 10c des Abzweigungselements 10 ist mit dem einen Ende einer ersten Ent- bzw. Belüftungsleitung 14 in Form eines flexiblen Schlauchleitungsstücks verbunden, die an ihrem freien Ende mit einer hydrophoben, d.h. gasdurchlässigen, aber für Flüssigkeiten undurchlässigen Membran 15 verschlossen ist.

Von dem Ent- bzw. Belüftungsanschluß 9 der zweiten Filterkammer 6 geht eine zweite Ent- bzw. Belüftungsleitung 16 in Form eines flexiblen Schlauchleitungsstücks ab, die an ihrem freien Ende ebenfalls mit einer hydrophoben Membran 17 verschlossen ist. Die beiden Endstücke der ersten und zweiten Ent- bzw. Belüftungsleitung 14, 16 sind in einem kollabierbaren Behältnis 18 in Form eines flexiblen Kunststoffbeutels eingeschweißt. Der Kunststoffbeutel weist an seinem oberen Rand eine Lasche 19 auf, durch die die erst Sammelleitung 11 geführt ist, so daß dieser an der Schlauchleitung befestigt ist (Figur 3).

Zum Öffnen und Verschließen der zweiten Ent- bzw. Belüftungsleitung 16 ist eine zweite Schlauchklemme 20 vorgesehen, die zwischen dem Kunststoffbeutel 18 und dem Ent- bzw. Belüftungsanschluß 9 auf der Schlauchleitung sitzt.

Das Sammelbehältnis 1 ist ein flexibler Kunststoffbeutel, der aus zwei an ihren Rändern miteinander verschweißten Kunststoffolien besteht. An dem oberen Rand des Kunststoffbeutels sind zwei mit durchstechbaren Membranen verschlossene Auslaßstutzen 21, 22 eingeschweißt, die jeweils mit aufreißbaren Laschen 23, 24 steril umschlossen sind. Die Anschlußstutzen 21, 22 dienen zur Entnahme des leukozytenfreien Thrombozytenkonzentrats mittels geeigneter Spike-Konnektoren.

Das Sammelbehältnis 1 weist darüberhinaus einen Einlaß 25 auf, der über eine zweite Sammelleitung 26 in Form einer flexiblen Schlauchleitung mit dem Auslaß 8 der zweiten Filterkammer 6 verbunden ist. Zum Öffnen und Schließen der zweiten Sammelleitung ist eine dritte Schlauchklemme 27 vorgesehen.

Nachfolgend wird das Verfahren zum Filtrieren von Flüssigkeiten, in diesem Ausführungsbeispiel zur Gewinnung eines leukozytenfreien Thrombozytenkonzentrats, unter Verwendung des Filtriersystems im einzelnen beschrieben.

Zunächst werden die Schlauchklemmen 12 und 20 der ersten Sammelleitung 11 und der zweiten Ent- bzw. Belüftungsleitung 16 geschlossen. Das die zu filtrierende Flüssigkeit, d.h. das Thrombozytenkonzentrat, enthaltende Behältnis, das in Figur 1 mit dem Bezugszeichen 28 versehen ist, wird nun an das Filtriersystem konnektiert. Zur Herstellung einer sterilen Verbindung wird das Behältnis 28 nicht mittels des Spike-Konnektors 13, sondern mittels einer sterilen Verbindungseinrichtung konnektiert, mit der das Behältnis stromauf des Filters 2 an die Sammelleitung 11 angeschlossen wird. Derartige sterile Verbindungseinrichtungen sind allgemein bekannt. Dabei bleibt die Verschlußkappe 13a auf dem Spike-Konnektor 13 aufgesteckt, der nur zur Herstellung einer Verbindung dient, bei der Luft von außen in das System eindringen kann. Derartige sterile Verbindungseinrichtungen gehören zum Stand der Technik und werden daher nicht weiter beschrieben. Daraufhin wird das Filtriersystem in vertikaler Richtung ausgerichtet, d.h. das Behältnis 28 befindet sich oberhalb des Sammelbehältnisses 1. Anschließend wird die Schlauchklemme 12 an der ersten Sammelleitung 11 wieder geöffnet, so daß die zu filtrierende Flüssigkeit unter Verdrängung der in der ersten Sammelleitung 11, dem Filter 2 und dem oberen Schlauchleitungsabschnitt der zweiten Sammelleitung 26 befindlichen Luft durch die hydrophoben Membranen 15, 17 der ersten und zweiten Ent- bzw. Belüftungsleitung in das Filtriersystem fließt. Die Flüssigkeit hört auf zu fließen, sobald diese mit der hydrophoben Membran 17 der zweiten Ent- bzw. Belüftungsleitung 16 in Kontakt kommt.

Damit ist das Filtriersystem befüllt. Um den Filtrationsvorgang zu starten, werden die Schlauchklemmen 20 und 27 an der zweiten Ent- bzw. Belüftungsleitung 16 bzw. der zweiten Sammelleitung 26 geöffnet. Das zu filtrierende Blut strömt nun über die erste und zweite Sammelleitung 11, 26 in das Sammelbehältnis 1. Nachdem das Behältnis 28 entleert ist, entleert sich auch die erste Filterkammer 5, ohne daß ein manueller Eingriff erforderlich ist. Dies ist darauf zurückzuführen, daß die zuvor in dem kollabierbaren Behältnis 18 gesammelte sterile Luft über die hydrophobe Membran 15 an der ersten Ent- bzw. Belüftungsleitung 14 in die erste Filterkammer 5 strömen kann.

Wenn die erste Filterkammer 5 nach etwa 60 Sekunden vollständig entleert ist, wird die an der zweiten Ent- bzw. Belüftungsleitung 16 vorgesehene Schlauchklemme 20 wieder geöffnet, so daß die zweite Filterkammer 6 mittels der in dem kollabierbaren Behältnis 18 noch befindlichen Luft, die durch die hydrophobe Membran 17 zuströmt, vollständig entleert werden kann. Die an der zweiten Sammelleitung 26 vorgesehene Schlauchklemme 27 wird rechtzeitig geschlossen, bevor die in dem Behältnis 28 befindliche Luft in das Sammelbehältnis 1 gelangt. Ist die Schlauchklemme 27 nicht rechtzeitig geschlossen worden, so wird das Sammelbehältnis 1 zusammengedrückt, so daß die in dem Sammelbehältnis befindliche Luft über die zweite Sammelleitung 26, den Filter 2, die zweite Ent- bzw. Belüftungsleitung 16 sowie die hydrophobe Membran 17 in das kollabierbare Behältnis 18 zurückströmt. Mit diesen Maßnahmen ist das Filtriersystem vollständig entleert.

## Patentansprüche

1. Steriles System zum Filtieren von Flüssigkeiten, insbesondere von Blut oder Blutkomponenten, mit
einem Filter (2), der ein Gehäuse (3) aufweist, das durch ein Filtermaterial (4) in zwei Kammern (5, 6) unterteilt ist, von denen die erste Kammer (5) mit einem Einlaß (7) und die zweite Kammer (6) mit einem Auslaß (8) versehen ist,
einer ersten mit dem Einlaß (7) der ersten Filterkammer (5) verbundenen Sammelleitung (11) zum Zuführen der zu filtrierenden Flüssigkeit,
einem Sammelbehältnis (1), das einen Einlaß (25) aufweist,
einer zweiten Sammelleitung (26), die den Auslaß (8) der zweiten Filterkammer (6) mit dem Einlaß (25) des Sammelbehältnisses (1) verbindet und
Mitteln (12) zum Öffnen bzw. Verschließen der ersten Sammelleitung (11) und Mitteln (27) zum Öffnen bzw. Verschließen der zweiten Sammelleitung (26),
einem ersten Luftein- bzw. -auslaß, der über eine erste Ent- bzw. Belüftungsleitung (14) mit der ersten Filterkammer (5) in Strömungsverbindung steht und einem zweiten Luftein- bzw. -auslaß, der über eine zweite Ent- bzw. Belüftungsleitung (16) mit der zweiten Filterkammer (6) in Strömungsverbindung steht, wobei der erste und zweite Luftein- und -auslaß in einem kollabierbaren Behältnis (18) angeordnet und Mittel (20) zum Öffnen bzw. Verschließen des zweiten Luftein- bzw. -auslaßes vorgesehen sind,
**dadurch gekennzeichnet,**
**daß** die zweite Ent- bzw. Belüftungsleitung (16) an der zweiten Filterkammer (6) angeschlossen ist, und daß der erste und zweite Luftein- bzw. -auslaß mit einer hydrophoben Membran (15, 17) verschlossen sind.

2. Steriles System nach Anspruch 1, **dadurch gekennzeichnet, daß** das kollabierbare Behältnis ein flexibler Kunststoffbeutel (18) ist.

3. Steriles System nach Anspruch 1 oder 2, **dadurch gekennzeichnet, daß** die erste Ent- bzw. Belüftungsleitung (14) von der ersten Sammelleitung (11) zwischen den Mitteln (12) zum Öffnen bzw. Verschließen der ersten Sammelleitung und dem Einlaß (7) der ersten Filterkammer (5) abzweigt.

4. Steriles System nach einem der Ansprüche 1 bis 3, **dadurch gekennzeichnet, daß** das kollabierbare Behältnis (18) eine Lasche (19) aufweist, durch die die erste Sammelleitung (11) geführt ist.

5. Steriles System nach einem der Ansprüche 1 bis 4, **dadurch gekennzeichnet, daß** am freien Ende der ersten Sammelleitung (11) ein Spike-Konnektor (13) angeschlossen ist.

6. Steriles System nach einem der Ansprüche 1 bis 5, **dadurch gekennzeichnet, daß** die Mittel zum Öffnen bzw. Verschließen der ersten und zweiten Sammelleitung (11, 26) und der zweiten Ent- bzw. Belüftungsleitung (16) auf den Leitungen sitzende Schlauchklemmen (12, 27, 20) sind.

7. Steriles System nach einem der Ansprüche 1 bis 6, **dadurch gekennzeichnet, daß** der Filter ein Leukozytenfilter (2) ist.

8. Verfahren zum Filtrieren von Flüssigkeiten, insbesondere von Blut oder Blutkomponenten, mit folgenden Verfahrensschritten:
Leiten der zu filtrierenden Flüssigkeit durch eine erste Sammelleitung (11) in die erste Kammer (5) eines durch ein Filtermaterial (4) in zwei Kammern (5, 6) unterteilten Filters (2), und aus der ersten Filterkammer (5) durch das Filtermaterial (4) in die zweite Filterkammer (6), an der eine zweite zu einem Sammelbehältnis (1) für die filtrierte Flüssigkeit führende Sammelleitung (26) angeschlossen ist, die mittels eines Sperrorgangs (27) verschlossen ist, wobei die in der ersten Filterkammer (5) befindliche Luft durch eine mit einer hydrophoben Membran (15) verschlossene erste Ent- bzw. Belüftungsleitung (14) und die in der zweiten Filterkammer (6) befindliche Luft durch eine mit einer hydrophoben Membran (17) verschlossene zweite Ent- bzw. Belüfungsleitung (16) in ein kollabierbares Behältnis (18) verdrängt wird,
Öffnen des Sperrorgangs (27) der zweiten Sammelleitung (26) zur Einleitung des Filtrationsvorganges, so daß die Filtrierflüssigkeit in das Sammelbehältnis (1) fließt und
nach Beendigung des Filtrationsvorganges Zurückführen der in dem kollabierbaren Behältnis (18) gesammelten Luft über die erste Ent- bzw. Belüftungsleitung (14) in die erste Filterkammer (5) und über die zweite Ent- bzw. Belüftungsleitung (16) in die zweite Filterkammer (6) unter Entleerung derselben.

9. Verfahren nach Anspruch 8, **dadurch gekennzeichnet, daß** die in dem kollabierbaren Behältnis (18) gesammelte Luft zunächst der ersten Filterkammer (5) unter Entleerung derselben zugeführt wird und daß dann die in dem kollabrierbaren Behältnis gesammelte Luft der zweiten Filterkammer (6) unter Entleerung derselben zurückgeführt wird.

## Claims

1. Sterile system for filtering liquids, especially blood or blood components, with a filter (2), possessing a housing (3) which is divided by a filtering material (4) into two chambers (5, 6) the first chamber of which (5) is provided with an inlet (7) and the second chamber (6) with an outlet (8),
a first collecting tube (11) connected to the inlet (7) of the first filter chamber (5) to feed in the liquid requiring to be filtered,
a collection container (1) which has an inlet (25),
a second collecting tube (26) connecting the outlet (8) of the second filter chamber (6) with the inlet (25) of the collecting chamber (1),
a means (12) for opening or closing the first collecting tube (11) and means (27) for opening or closing the second collecting tube (26),
a first inlet- or outlet for air which is flow-connected by a first extraction- or supply tube for air (14) with the first filter chamber (5) and a second inlet or outlet for air which is flow-connected by a second extraction or supply tube for air (16) with the second filter chamber (6) whereby the first and second air inlet and air outlet are arranged in a collapsible container (18) and means (20) to open or close the second air inlet or air outlet are provided,
**characterised in that** the second extraction or feed tube for air (16) is connected to the second filter chamber (6) and that the first and second air inlet or air outlet is closed off by means of a hydrophobic membrane (15, 17).

2. Sterile system in accordance with Claim 1, **characterised In that** the collapsible container consists of a flexible plastic bag (18).

3. Sterile system in accordance with Claim 1 or 2, **characterised in that** the first extraction or supply tube for air (14) branches off from the first collecting tube (11) between the means (12) for opening or closing the first collecting tube and the outlet (7) of the first filter chamber (5).

4. Sterile system in accordance with one of the Claims 1 to 3, **characterised in that** the collapsible container (18) possess an eye (19) through which the first collecting tube (11) passes.

5. Sterile system in accordance with one of the Claims 1 to 4, **characterised in that** a spike connector (13) is connected to the free end of the first collecting tube (11).

6. Sterile system in accordance with one of the Claims 1 to 5, **characterised in that** the means for opening or closing the first and second collecting tubes (11, 16) and the second extraction tube or supply tube for air (16) consist of hose clips fitted to the tubes.

7. Sterile system in accordance with one of the Claims 1 to 6, **characterised in that** the filter is a leukocyte filter (2).

8. Procedure for filtering liquids, especially of blood or blood components by means of the following procedural stages:
Conduction of the liquid to be filtered through a first collecting tube (11) into the first chamber (5) of a filter (2) divided by a filtering material into two chambers (5, 6) and out of the first filter chamber (5) through the filter material (4) into the second filter chamber (6) to which is connected a second collecting tube (26) leading to a collecting container (1) for the filtered liquid to which is attached a second collecting tube (26) which is closed off by a clamping device (27), whereby the air in the first filter chamber (5) is forced through a first extraction or supply tube for air (14) closed off by a hydrophobic membrane (15) and the air in the second filter chamber (6) is forced through a second extraction or supply tube for air (16) closed off by a hydrophobic membrane into a collapsible container (18),
Opening the clamping device (27) on the second collecting tube (26) to initiate the filtering procedure so that the filtered liquid flows into the collecting container (1) and
after the conclusion of the filtering procedure the return flow of the air collected in the collapsible container (18) through the first extraction or supply tube for air (14) into the first filter chamber (5) and through the second extraction or supply tube for air (16) into the second filter chamber (6) brought about by the emptying of that chamber.

9. Procedure in accordance with Claim 8, **characterised in that** the air collected in the collapsible container (18) is first fed into the first filter chamber (5) as it empties and then the air collected in the collapsible container is fed into the second filter chamber (6) as it empties.

## Revendications

1. Système stérile pour la filtration de liquides, en particulier de sang ou de composants sanguins, avec
un filtre (2), qui présente un boîtier (3), qui est partagé en deux chambres (5, 6) par un matériau de filtre (4), parmi lesquelles la première chambre (5) est munie d'une entrée (7) et la deuxième chambre (6) d'une sortie (8),
une première canalisation globale (11) liée à l'entrée (7) de la première chambre de filtre (5) pour l'alimentation du liquide à filtrer,
un récipient de récupération (1), qui présente une entrée (25),
une deuxième canalisation globale (26), qui relie la sortie (8) de la deuxième chambre de filtre (6) à l'entrée (25) du récipient de récupération (1) et
des moyens (12) pour ouvrir ou fermer la première canalisation globale (11) et des moyens (27) pour ouvrir ou fermer la deuxième canalisation globale (26),
une première entrée et sortie d'air, qui est reliée avec la première chambre de filtration (5) à travers une première canalisation de désaération ou d'aération (14) et une deuxième entrée ou sortie d'air, qui est reliée avec la deuxième chambre de filtration (6) à travers une deuxième canalisation de désaération ou d'aération (16), dans lequel la première et la seconde entrée et sortie d'air sont disposées dans un récipient (18) pouvant s'affaisser et sont munies de moyens (20) pour ouvrir ou fermer la deuxième entrée ou sortie d'air,
**caractérisé en ce que**,
la deuxième canalisation de désaération ou d'aération (16) est reliée à la deuxième chambre de filtration (6), et **en ce que** la première et deuxième entrée ou sortie d'air sont fermées par une membrane hydrophobe (15, 17).

2. Système stérile selon la revendication 1, **caractérisé en ce que** le récipient pouvant s'affaisser est un sac de matière plastique flexible (18).

3. Système stérile selon la revendication 1 ou 2, **caractérisé en ce que** la première canalisation de désaération ou d'aération (14) est ramifiée de la première canalisation globale (11) entre les moyens (12) pour l'ouverture ou la fermeture de la première canalisation globale et l'entrée (7) de la première chambre de filtration (5).

4. Système stérile selon l'une des revendications 1 à 3, **caractérisé en ce que** le récipient pouvant s'affaisser (18) présente un collier de fixation (19), à travers lequel la première canalisation globale (11) est guidée.

5. Système stérile selon l'une des revendications 1 à 4, **caractérisé en ce qu'**à l'extrémité libre de la première canalisation globale (11) est relié un connecteur-perforateur (13).

6. Système stérile selon-l'une des revendications 1 à 5, **caractérisé en ce que** les moyens pour l'ouverture et la fermeture de la première et de la deuxième canalisation globale (11, 26) et de la première ou deuxième canalisation d'aération (16) sont des pinces pour tuyaux souples (12, 27, 20) sur les canalisations.

7. Système stérile selon l'une des revendications 1 à 6, **caractérisé en ce que** le filtre est un filtre à leucocytes (2).

8. Procédé de filtration de liquides, en particulier de sang ou de composants sanguins, avec les étapes suivantes de procédé :
alimentation du liquide à filtrer à travers la première canalisation globale (11) dans la première chambre (5) d'un filtre (2) divisé en deux chambres (5, 6) au moyen d'un matériau filtrant, et de la première chambre de filtre (5) à travers le matériau filtrant (4) dans la deuxième chambre de filtre (6), à laquelle est reliée une deuxième canalisation globale (26) conduisant à un récipient de récupération pour le liquide filtré, qui est fermée au moyen d'un organe d'occlusion (27), dans lequel l'air se trouvant dans la première chambre de filtre (5) à travers une première canalisation de désaération ou d'aération (14) fermée par une membrane (15) hydrophobe et l'air se trouvant dans la deuxième chambre de filtre (6) à travers une deuxième canalisation de désaération ou d'aération (16) fermée par une membrane (17) hydrophobe est déplacé dans un récipient (18) pouvant s'affaisser,
ouverture de l'organe de fermeture (27) de la deuxième canalisation globale (26) pour l'introduction du produit de filtration, de manière à ce que le liquide de filtration d'écoule dans le récipient de récupération (1) et
après la fin de l'étape de filtration récupération de l'air rassemblé dans le récipient (18) pouvant être affaissé à travers la première canalisation de désaération ou d'aération (14) dans la première chambre de filtre (5) et à travers la deuxième canalisation de désaération ou d'aération (16) dans la deuxième chambre de filtre (6) en vidant celle-ci.

9. Procédé selon la revendication 8, **caractérisé en ce que** l'air collecté dans le récipient (18) pouvant être affaissé est d'abord introduit dans la première chambre de filtre (5) par vidange de celui-ci et qu'ensuite l'air rassemblé dans le récipient pouvant être affaissé est réintroduit dans la deuxième chambre de filtre (6) par vidange de celui-ci.
